# EUROPEAN PATENT SPECIFICATION

(11) **EP 2 812 050 B1**
(45) Date of publication and mention of the grant of the patent: **09.01.2019**
(21) Application number: 13746115.8
(22) Date of filing: 07.02.2013
(51) Int. Cl.: A61B 46/00, A61B 34/20

(54) **THREE-DIMENSIONAL GUIDED INJECTION DEVICE**
DREIDIMENSIONALE GEFÜHRTE INJEKTIONSVORRICHTUNG
DISPOSITIF D'INJECTION À GUIDAGE TRIDIMENSIONNEL

(30) Priority: 07.02.2012 US 201261595998 P
(43) Date of publication of application: 17.12.2014
(60) Divisional application: 18208394.9
(73) Proprietor: Joint Vue, LLC, Columbus OH 43212-1155 (US)
(72) Inventor: WASIELEWSKI, Ray, C., New Albany, OH 43054 (US)
(74) Representative: HGF Limited
(86) International application number: PCT/US2013/025131
(87) International publication number: WO 2013/119801

(56) References cited:
- GB-A- 2 400 176
- US-A- 6 122 541
- US-A1- 2004 147 839
- US-A1- 2006 264 751
- US-A1- 2009 012 509
- US-A1- 2010 100 011
- US-A1- 2010 198 067
- US-A1- 2011 054 303
- US-A1- 2011 054 303

## Description

### TECHNICAL FIELD

The present invention relates generally to therapeutic injection systems and, more particularly, to guided needle injection systems for injecting joints.

### BACKGROUND

Joint pain is a major public health problem and is responsible for significant costs and disability in the United States. This is due, at least in part, to underlying osteoarthritis. Joint pain occurs in approximately 46 million Americans and is increasing due to an aging population and an epidemic of increasing obesity. Joint pain costs the healthcare system about $37 billion annually. Depending on the degree of patient disability, joint pain can be treated with a range of systemic and targeted interventions.

One common method of treating joint pain is with injections. Joint injections are a medical procedure in which a hypodermic needle is inserted into an affected joint, and a pharmaceutical agent (for example, corticosteroids or anesthetics) is delivered to the joint through the needle. Joints should be injected under sterile conditions to prevent infections. To this end, a sterile prep sheet, or drape is commonly used in conjunction with sterilization of the injection site. During the injection, the sterile injection site is exposed by a window in the drape while the surrounding areas are covered to provide an expanded sterile field from the surrounding non-sterilized region.

Proper placement of the needle and pharmaceutical agent within the joint are required to provide the desired therapeutic effect when performing injection procedures. However, because injections are typically performed without needle guidance assistance, delivery accuracy depends entirely on the skill of the physician. Studies have revealed injection inaccuracies ranging from 18-34% in the knee and 33-90% in the shoulder, with similar missed injection rates in the hip. Therefore, there exists a need for devices for providing needle guidance, in real time, during an injection procedure to reduce the level of skill required to perform the injection, and to improve the accuracy of the injection.

Documents US 2009/0012509 A1, US 6,122,541 A, US 2011/0054303 A1 and US 2004/0147839 A1 disclose various navigation systems for guiding and/or tracking surgical instruments.

### SUMMARY

In an embodiment of the invention, a system is provided for guiding a needle during an injection. The system includes a drape configured to be positioned over a joint of a patient, the joint having an orientation. The system further includes one or more sensors coupled to the drape and configured provide signals indicative of the orientation of the joint.

### BRIEF DESCRIPTION OF THE FIGURES

FIG. 1 is a perspective view of an injection suite showing a patient prepped for an injection with a drape positioned over a joint of the patient, and an ultrasonic imaging system including a computer.
FIG. 2 is a diagrammatic view of the drape and the ultrasonic imaging system of FIG. 1.
FIG. 3 is a front view of a 3-D representation of the patient's joint of FIG. 1, and an outline showing the position of the drape with respect to the patient's joint.
FIG. 4 is a transparent front view of the patient's actual joint and the drape of FIG. 1 showing sensors embedded in the drape and the drape positioned on the joint so that the joint is ready to receive an injection.
FIG. 5 is a front view of a 3-D representation of a patient's hip joint and an outline showing the position of the drape with respect to the patient's hip joint.
FIG. 6 is a transparent front view of the patient's actual hip joint and the drape, showing sensors embedded in the drape and the drape positioned on the joint so that the joint is ready to receive the injection.
FIG. 7 is the transparent front view of the patient's actual hip joint of FIG. 6 further illustrating an ultrasound probe for monitoring and re-localization of the needle relative to the joint.
FIG. 8 is a front view of a 3-D representation of a patient's shoulder joint and an outline showing the position of a drape with respect to the patient's shoulder joint.
FIG. 9 is a transparent front view of the patient's actual shoulder joint and the drape showing sensors embedded in the drape and the drape positioned on the joint so that the joint is ready to receive the injection.
FIG. 10 is a rear view of a 3-D representation of a patient's lumbar spine and an outline showing the position of a drape with respect to the patient's lumbar spine.
FIG. 11 is a transparent rear view of the patient's actual lumbar spine and the drape, showing EMU sensors embedded in the drape and the drape positioned on the lumbar spine for injection into the lumbar spine.
FIG. 12 is a transparent rear view of the patient's actual lumbar spine and an alternative of the drape in FIG. 11 including a plurality of regions having ultrasound sensors embedded with different densities.
FIG. 13 is a transparent rear view of the patient's actual lumbar spine and an alternative of the drape of FIG. 12 having a plurality of windows exposing injections sites on the lumbar.
FIG. 14 is a transparent rear view of the patient's actual spinal lumbar and an alternative of the drape of FIG. 13 having a plurality of windows exposing injections sites on the lumbar.

### DETAILED DESCRIPTION

The present invention addresses the foregoing problems and other shortcomings, drawbacks, and challenges of conventional joint injection protocols by providing a drape that facilitates real-time registration, or location of an injection needle relative to the joint and bones, for aiding guidance of the needle into the joint. The drape is configured to provide a sterile filed and includes one or more windows through which injections can be made. The drape also includes a plurality of sensors that facilitate locating the injection needle within the joint being injected. In an embodiment of the invention, the sensors include ultrasound sensors configured to be operatively coupled to an imaging system. The imaging system may thereby provide real-time images of the joint to aid in guiding the injection needle based on signals received from the ultrasound sensors in the drape. The drape also includes, in addition to the ultrasound sensors, one or more Inertial Measurement Unit (IMU) sensors for detecting motion of the joint. The detected motion may in turn be used by the imaging system to adjust the orientation of a three-dimensional (3-D) joint representation, or model, stored in memory from a previous registration of the 3-D joint representation to the joint.

By providing the attending physician with real-time images of the joint during an injection, embodiments of the invention provide the physician with feedback regarding the movement and position of the needle with respect to the joint as the injection procedure is being performed. The joint images thus facilitate placing the tip of the injection needle more precisely into an optimal position within the joint. This optimal needle placement helps ensure that the injected material is delivered in a desired location within the joint. Needle injection using real-time ultrasound guidance and inertial position measurement with 3-D joint visualization may thereby improve injection accuracy, reduce time spent on joint injections, reduce the cost and complexity of the process, and reduce the pain and discomfort to patients caused by multiple or missed injection attempts.

Although the embodiments of the invention described herein are focused on knee, hip, shoulder, and spinal joint injections, persons having ordinary skill in the art will recognize that other joint injections could also benefit from 3-D guidance. These joints include, but are not limited to the sacroiliac, elbow, wrist, feet, neck, and hands, for example. Moreover, persons having ordinary skill in the art will further understand that bursae, tendon, and other musculoskeletal and soft tissue injections could similarly benefit from real-time guidance.

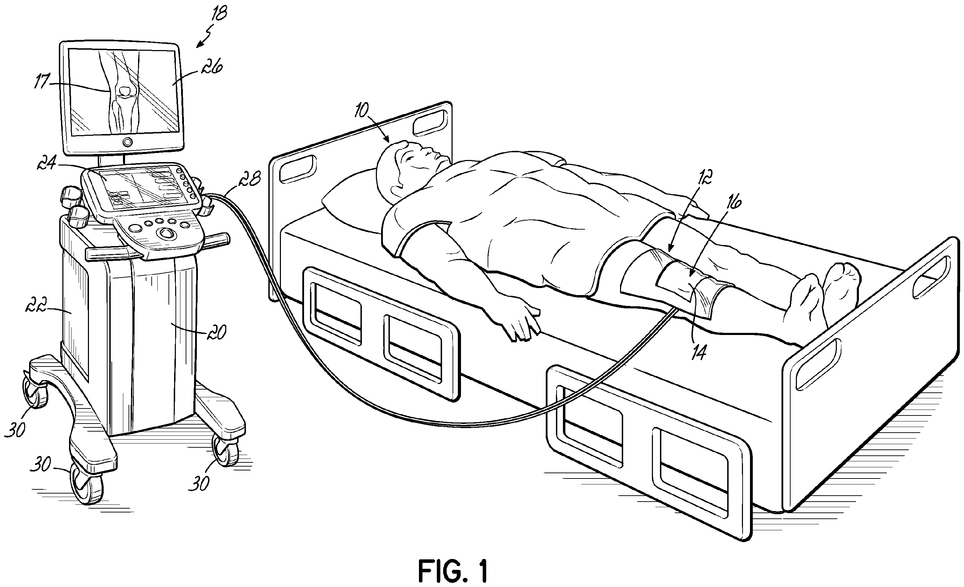

Referring now to FIG. 1, a patient 10 is shown in an injection suite with a drape 12 including a window 14 that is placed over a joint 16 (e.g., a knee) of patient 10. The window 14 provides an opening in the drape 12 through which an injection into the joint 16 can be made. The window 14 typically comprises an opening in the drape 12, but may also include a transparent or semi-transparent material, such as an adhesive backed film, that covers the opening and adheres to the patient's skin for injection through the film. Indeed, the window 14 may be comprised of any suitable material through which an injection can be made, but preferably a material that is transparent to ultrasound. To this end, the window 14 may be comprised of a section of the material comprising the drape 12 that is free of obstructions that could otherwise interfere with passage of the injection needle.

Typically, an ultrasound imaging system 18 will be located in the injection suite. The ultrasound system 18 may be used to obtain ultrasonic data that is used to provide an image 17 of the joint 16 to the attending physician (not shown) during the injection procedure. To this end, the ultrasound imaging system 18 may be configurable to access acquired raw RF ultrasound data, or pulse echo signals for processing by a computer 22. One suitable instrument may include, for example, the diagnostic ultrasound model SonixRP by Ultrasonix Inc. (Richmond, British Columbia, Canada). The ultrasound imaging system 18 includes a housing 20 containing a controller, (e.g., the computer 22), an energy or power source (not shown), a user input device 24, an output device (e.g., a touchscreen or monitor 26), and one or more cables 28 for coupling the ultrasound imaging system 18 to the drape 12. The coupling connection between the computer 22 and drape 12 might also be wireless and handled by a suitable wireless connection, such as an ultra-wideband link. The housing 20 may also include caster wheels 30 to facilitate transporting the ultrasound imaging system 18 between multiple injection suites in a treatment facility. Although shown in a supine position, the patient 10 may also be in an elevated position. In addition, a leg positioner (not shown) may be used to raise the joint 16 so that it is positioned at an angle suitable for performing the injection.

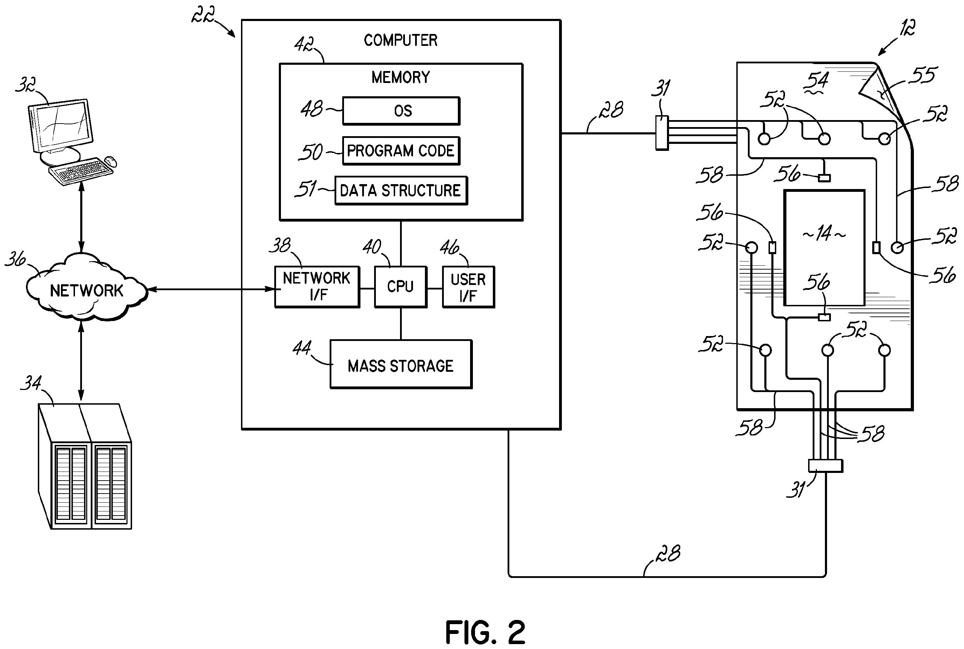

Referring now to FIG. 2, the computer 22 of ultrasound imaging system 18 is shown coupled to the drape 12 by the one or more cables 28, which may connect to the drape 12 via corresponding connectors 31. The computer 22 may represent any type of computer, computer system, computing system, server, disk array, or programmable device such as multi-user computers, single-user computers, handheld devices, networked devices, or embedded devices, etc. The computer 22 may be implemented with one or more networked computers 32 or networked storage devices 34 using one or more networks 36, e.g., in a cluster or other distributed computing system through a network interface 38 (illustrated as "NETWORK I/F"). For brevity's sake, the computer 22 will be referred to simply as "computer," although it should be appreciated that the term "computing system" may also include other suitable programmable electronic devices consistent with embodiments of the present invention.

The computer 22 includes a Central Processing Unit (CPU) 40 coupled to a memory 42 along with several different types of peripheral devices, e.g., a mass storage device 44, a user interface 46 (illustrated as "User I/F"), which may include the input device 24 and the monitor 26, and the Network I/F 38. The memory 42 may include dynamic random access memory ("DRAM"), static random access memory ("SRAM"), non-volatile random access memory ("NVRAM"), persistent memory, flash memory, one or more hard disk drives, and/or other digital storage mediums. The mass storage device 44 typically includes at least one hard disk drive and may be located externally to the computer 22, such as in a separate enclosure, in one or more of the networked computers 32, or one or more of the networked storage devices 34 (for example, in a database server).

The CPU 40 may be a single-thread, multi-threaded, multi-core, and/or multi-element processing unit. In alternative embodiments, the computer 22 may include a plurality of processing units that may include single-thread processing units, multi-threaded processing units, multi-core processing units, multi-element processing units, and/or combinations thereof. Similarly, the memory 42 may include one or more levels of data, instruction, and/or combination caches, with caches serving the individual processing unit or multiple processing units.

The memory 42 of computer 22 may include an operating system 48 (illustrated as "OS"), program code 50, and one or more data structures 51. The operating system 48 may be configured to manage computer resources so that the program code 50 in memory 42 may have instructions executed by the CPU 40. The program code 50 may represent computer instructions that provide at least one application, component, algorithm, program, object, module, or sequence of instructions to the CPU 40. Program code 50 typically comprises one or more instructions that are resident at various times in the memory 42 and/or the mass storage device 44 of the computer 22, and that, when read and executed by the CPU 40, causes the computer 22 to perform the steps necessary to execute steps or elements embodying the various aspects of the present invention. The one or more data structures 51 may be used by the CPU 40, operating system 48, and/or program code 50 to store or register data, such as data representing 3-D joint representations, models, images, and/or sensor data.

The illustrated embodiment of drape 12 includes a plurality of ultrasound transducers or sensors 52, and a plurality of IMU sensors 56. The ultrasound sensors 52 and IMU sensors 56 may be coupled to the one or more connectors 31 by wires 58. The connectors 31 thereby facilitate operatively coupling the sensors 52, 56 to the ultrasound imaging system 18 and/or computer 22 via the one or more cables 28. The ultrasound sensors 52 may be either static or dynamic. That is, the sensors 52 may be fixed or moveable with respect to the drape 12. In an embodiment of the invention, the ultrasound sensors 52 are round sensors including one or more elements, i.e., a single element or multiple elements. The ultrasound sensors 52 including multiple elements may be comprised of any number of elements arranged in any design or pattern.

In an alternative embodiment of the invention, the IMU sensors 56 may be coupled to the computer 22 using a wireless link. The drape 12 may also include data processing circuitry (e.g., CPU 40 or similar processor) to process signals received from the IMU sensors 56, and an ultra-wide band or other suitable transmission circuit to communicatively couple the IMU sensors 56 and/or processing circuitry to the computer 22. The processing circuitry may facilitate communication over the wireless link by converting the signals generated by the IMU sensors 56 into a form more suitable for wireless transmission, such a digital signal indicative of the relative motion of the IMU sensors 56.

Embodiments of the drape 12 include one or more single element and/or multiple element round ultrasound sensors 52 positioned on or embedded in the drape 12. In embodiments of the drape 12 that include a plurality of ultrasound sensors 52, the sensors 52 may be distributed in the drape 12 with a generally uniform density, although varying densities may also be used. One or more of the ultrasound sensors 52 may also include a linear array of sensor elements positioned on at least a portion of drape 12 to provide higher resolution ultrasound imaging.

The one or more IMU sensors 56 are included in addition to the one or more ultrasound sensors 52. When using an embodiment of the drape 12 the sensors 56 may be monitored by the computer 22 to track changes in the position of the joint 16 based on sensed movement of the patient 10, as will be described in more detail below.

In embodiments of the invention having a large number of sensors 52, 56, ultrasound connectors 31 and/or multi-pin connectors 31 having a high pin density may be used to couple the drape 12 to the one or more cables 28. By way of example only, one or more connectors 31 each including 256 discrete ultrasound connections may be used to provide separate connections to each of the ultrasound sensors 52 and/or sensor elements of the drape 12. In particular, one or more connectors 31 having a high pin density may be used if the drape 12 has a high density of ultrasound sensors 52, and/or the individual sensors 52 include a large number of elements.

The drape 12 may be constructed from a sheet of suitable material having a top surface 54 configured to face outward from the patient 10 and a bottom surface 55 configured to contact the skin of the patient 10. In an embodiment of the invention, the bottom surface 55 may be tacky and/or include an adhesive so that the drape 12 is held in place on the patient 10. In embodiments including an adhesive, the adhesive portion of the drape 12 may be a separate component (e.g., a removable sheet) so that the sensor containing portion of the drape 12 may be re-used after the adhesive portion is disposed of. In any case, the sheet material may also be transparent enough for the treating physician to see the skin of patient 10 through the drape 12, at least to some degree.

The sheet may also preferably be of a material with an acoustic impedance suitable for transmitting ultrasound between the patient 10 and an ultrasound probe in contact with the top surface 54 of drape 12, as well as between the patient 10 and the ultrasound sensors 52 in the drape 12. One such suitable material is a silicone based material, such as an ALPHA silicone liner having a thickness ranging from about 0,159 cm (1/16 inch) to about 0,635 cm (1/4 inch) that is available from WILLOWWOOD (The Ohio Willow Wood Company, Inc., Mt. Sterling, Ohio). Such materials allow the physician to perform an ultrasound scan, image the joint 16, and inject a needle into the joint 16 directly through the drape 12. In an alternative embodiment of the invention, the silicone liner material may be used for the window 14, and may be positioned within another material to form a laminate comprising the rest of the drape 12. In any case, the window 14 is configured to provide a sterile field for the injection. The window 14 is also configured to provide an area for rescanning of the joint 16 if necessary to re-localize the position of the bones comprising the joint 16, for example, in response to patient movement.

Those skilled in the art will recognize that the environment illustrated in FIG. 2 is for exemplary purposes only, and is not intended to limit embodiments of the invention. Indeed, those skilled in the art will recognize that embodiments of the invention may be used in other environments to facilitate injections, and environments including alternative hardware and/or software environments may be used without departing from the scope of embodiments of the invention. For example, and as described in more detail below, other embodiments of the invention may include drapes configured to facilitate injections of other joints. Moreover, embodiments of the invention may include drapes used for injections in a veterinary setting on non-human subjects, such as dogs, cats, race horses, farm and zoo animals, or any other animal being given a guided injection.

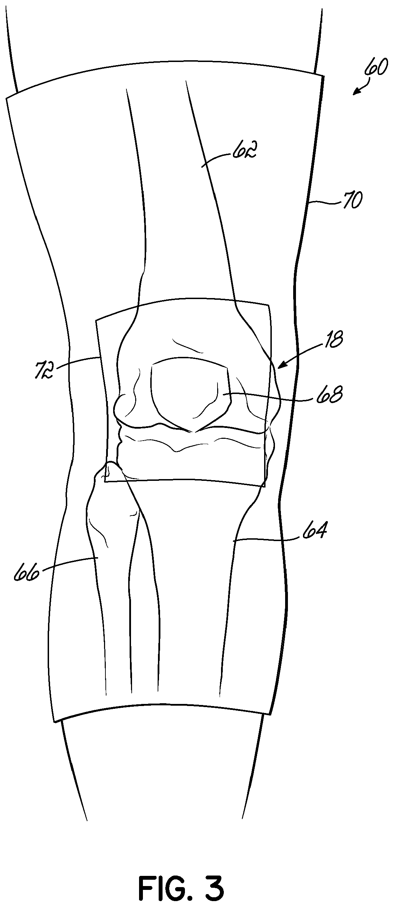

Referring now to FIG. 3, a front view of a 3-D representation 60 of the joint 16 is illustrated showing a femur 62, a tibia 64, a fibula 66, and a patella 68. The location of the drape 12 relative to the 3-D representation 60 is indicated by outlines 70, 72, with the window 14 of drape 12 positioned over the joint 16 as illustrated by the outline 72. The outlines 70, 72 thereby show a typical position of the drape 12 relative to the joint 16 during an injection procedure. The 3-D representation 60 may be a digital model of the joint 16 accessible by the computer 22, and may be generated using any suitable medical imaging modality and stored as a digital file prior to the injection procedure. These imaging modalities may include any suitable imaging technology, such as Computed Tomography (CT), Magnetic Resonance Imaging (MRI), and/or 3-D ultrasound reconstruction.

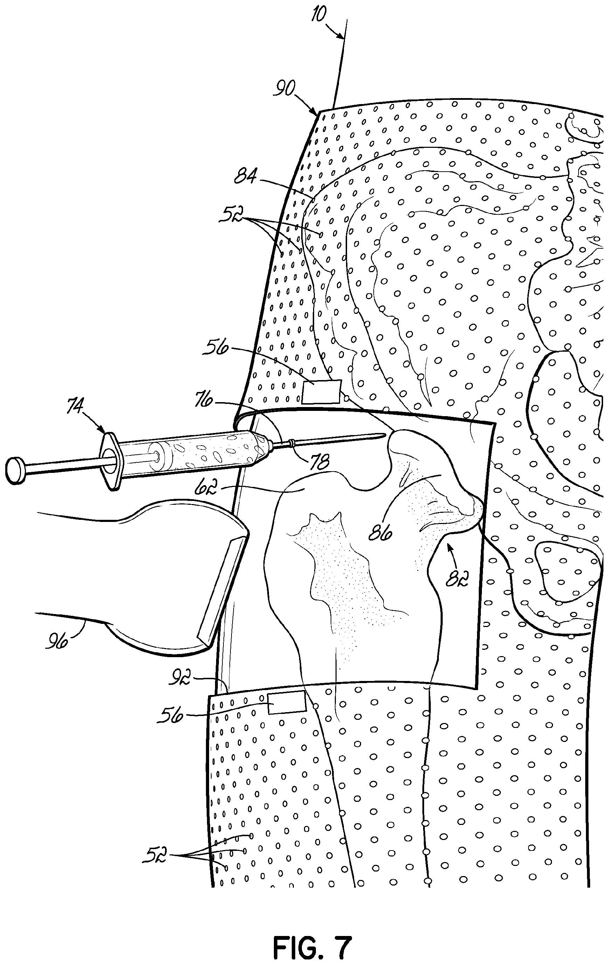

In an embodiment of the invention, the 3-D representation 60 of the joint 16 (or other ligament or tendon, as the case may be) is registered with the joint 16 by collecting ultrasound signal data, such as with an ultrasound probe 96 (FIG. 7), before the drape 12 is placed on the patient 10. That is, ultrasound signal data may be collected and processed to obtain a plurality of bone contours from the joint 16 prior to drape placement. This bone contour data may be used in combination with position data collected for the ultrasound probe 96 during acquisition of the bone contours to determine the positions of the bones 62, 64, 66, 68 of joint 16. The probe position data collected during acquisition of the bone contours may be generated by a suitable position tracking system, such as an optical or electromagnetic tracking system as is known in the art. The bone positions determined from the ultrasound signal data may in turn be used to adjust the orientation of the 3-D representation 60 to match that of the joint 16.

By scanning the joint 16 with the ultrasound probe 96 prior to placing the drape 12 on the patient 10, fine details of the joint 16 (such as spine facets in the case of the vertebrae), may be reconstructed. Thus, for more complex joint or tendon configurations, it may be desirable to generate a fine detailed 3-D representation of the desired injection region prior to drape placement. In the case of joints having large joint spaces, such as a knee joint, this prior 3-D scanning may not be necessary. That is, registration of the 3-D representation 60 may be accomplished by relying on the ultrasound sensors 52 in the drape 12. In this case, ultrasound data may be collected from the joint 16 by the ultrasound sensors 52, and the 3-D representation 60 registered to the joint 16 after the drape 12 has been placed on the patient 10.

The 3-D representation 60 may be stored as one or more data structures 51 in memory 42 of computer 22 so that the representation data can be accessed by the CPU 40 as needed while executing the program code 50. The 3-D representation 60 may thereby be used by the computer 22 to generate the image 17 displayed on the monitor 26. In cases where the 3-D representation 60 is generated using 3-D ultrasound reconstruction, the 3-D representation 60 may be generated contemporaneously with the injection procedure using the ultrasound sensors 52 of drape 12. The outlines 70, 72 may also be superimposed over the image 17 by the computer 22 to provide a visual reference point for the attending physician while performing the injection.

Exemplary methods of generating the 3-D representation 60 of the joint 16 are described in more detail in pending International Application No. PCT/US11/46318, filed on August 2, 2011, and entitled METHOD AND APPARATUS FOR THREE DIMENSIONAL RECONSTRUCTION OF A JOINT USING ULTRA-SOUND (Pub. No WO 2012/018851), in pending International Application No. PCT/US11/54952, filed on October 5, 2011, and entitled METHOD AND APPARATUS FOR THREE-DIMENSIONAL RECONSTRUCTION OF JOINT USING ULTRASOUND (Pub. No. WO 2012/048020), and in pending International Application No. PCT/US12/60261, filed on October 15, 2012, and entitled REAL-TIME 3-D ULTRASOUND RECONSTRUCTION OF KNEE AND ITS IMPLICATIONS FOR PATIENT SPECIFIC IMPLANTS AND 3-D JOINT INJECTIONS (Pub. No. WO 2013/056231).

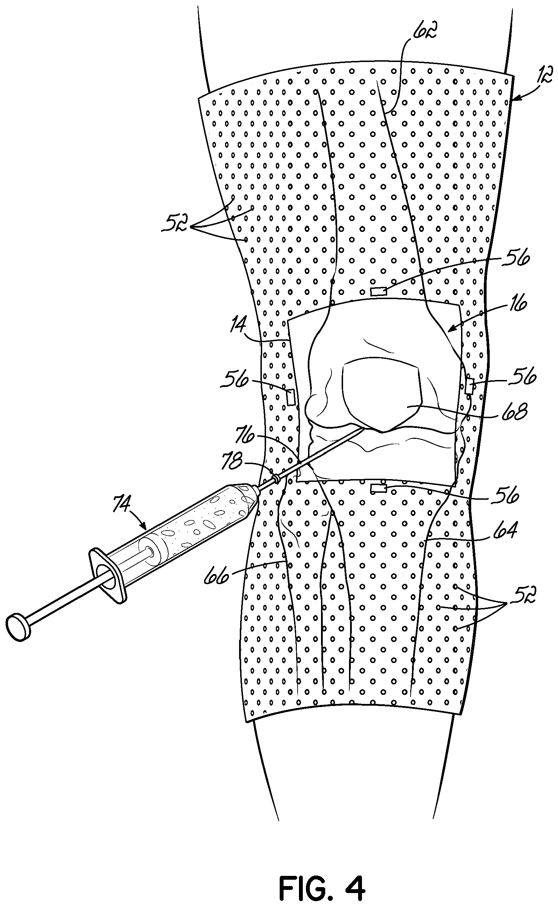

Referring now to FIG. 4, a transparent view of the patient's actual joint 16 is illustrated showing the drape 12, the bones 62, 64, 66, 68 comprising the joint 16, and a syringe 74 including a needle 76. The drape 12 is positioned over the joint 16 in accordance with the positioning shown by the outline of the drape 12 in FIG. 3 so that the window 14 is over the injection site. The drape 12 provides a dual functionality by: (1) providing a sterile field for the injection; and (2) containing one or more sensors 52, 56 configured to provide signals for determining the relative positions of the bones 62, 64, 66, 68 of joint 16 and the needle 76. In the illustrated embodiment, the drape 12 includes a plurality of ultrasound sensors 52 having a relatively uniform density throughout the portion of the drape 12 not including the window 14, and a plurality of IMU sensors 56 positioned peripherally around the window 14.

In an embodiment of the invention, the ultrasound imaging system 18 is operatively coupled to the ultrasound sensors 52 by the one or more cables 28 to obtain ultrasound imaging data from the joint 16. This ultrasound imaging data is provided to the computer 22, which detects and registers features of the joint 16 to corresponding features in the 3-D representation 60 of joint 16. The computer 22 may then generate and display the image 17 on the monitor 26 in real-time based on the 3-D representation 60 of joint 16. By altering the orientation of the 3-D representation 60 so that the positions of the features in the 3-D representation 60 conform to the determined positions of the features in the actual joint 16, the displayed image 17 may provide real-time guidance to the physician. That is, the image 17 may thereby represent the relative positions of the bones 62, 64, 66, 68 of joint 16 in real-time as the injection procedure is performed.

The location of the needle 76 may be determined based on the ultrasound imaging data obtained from the joint. However, in an alternative embodiment of the invention, the syringe 74 may include an electromagnetic tracking element 78 at an assigned location so that the needle tip position relative to the tracking element 78 is known and fixed. In systems that use an electromagnetic tracking element 78 to determine the position of the needle 76, an electromagnetic transceiver unit (not shown) may provide electromagnetic tracking element position data to the computer 22. The computer 22 may then display the position of the needle 76 relative to the joint 16 based on the data received from the electromagnetic transceiver unit. In an alternative embodiment of the invention, the needle 76 may include an echogenic feature (not shown) at a known distance from the tip of the needle 76. This echogenic feature may allow the computer 22 to determine the position of the tip based on a strong ultrasound reflection from the feature, or to simply to show a highlighted region of the needle 76 on the display 26.

In the embodiments of the invention, the IMU sensors 56 are configured to track patient movement during the injection procedure. This movement may be detected by the computer 22 based on signals received from the IMU sensors 56, and used to update the 3-D representation 60 of the joint 16 relative to the needle 76. In the illustrated embodiment, the IMU sensors 56 are placed along the medial, lateral, superior, and inferior portions of the drape 12 proximate the joint 16 so that if movement occurs, the bones 62, 64, 66, 68 of joint 16 may still be tracked relative to the incoming needle 76. The computer 22 may then generate the image 17 of joint 16 based on the updated 3-D representation 60 of joint 16 in a similar manner as described above. For example, if the joint 16 is registered to the 3-D representation 60 before drape placement as described above, the drape 12 may locate the bones in their initial positions. The IMU sensors 56 may then sense motion, which can be quantified to re-localized the bones 62, 64, 66, 68 of joint 16 so that the 3-D representation 60 may be reconstructed to represent the bones in their new locations with respect to the needle 76. The IMU sensors 56 may thereby eliminate the need to completely reconstruct the 3-D representation 60 or re-register the joint 16 with either the ultrasound probe 96 or the ultrasound sensors 52.

The image 17 of joint 16 displayed on the monitor 26 may be based on the 3-D representation 60 to provide the physician performing the injection procedure with feedback regarding the position of the needle 76 relative to the joint 16. The image 17 displayed on the monitor 26 may also be generated directly from ultrasound signals received from the sensors 52 of drape 12. In either case, the visual feedback provided by the image 17 of joint 16 may help the physician locate the needle 76 relative to the bones 62, 64, 66, 68 and/or other tissue comprising the joint 16 as the injection procedure is performed. By helping the physician locate the needle 76 accurately relative to the joint 16, embodiments of the invention may help the physician optimize the injection location.

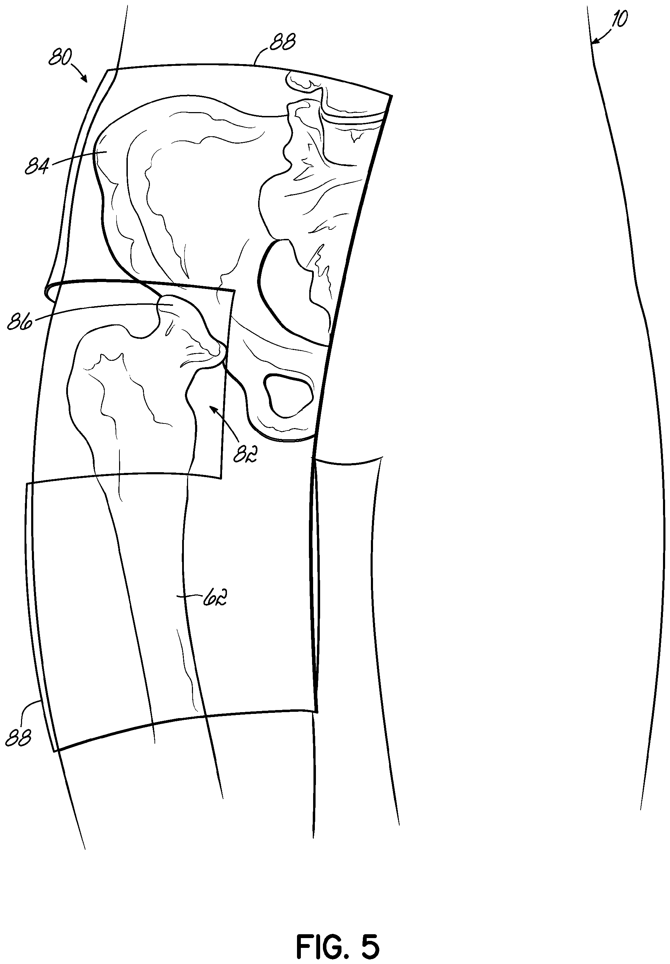

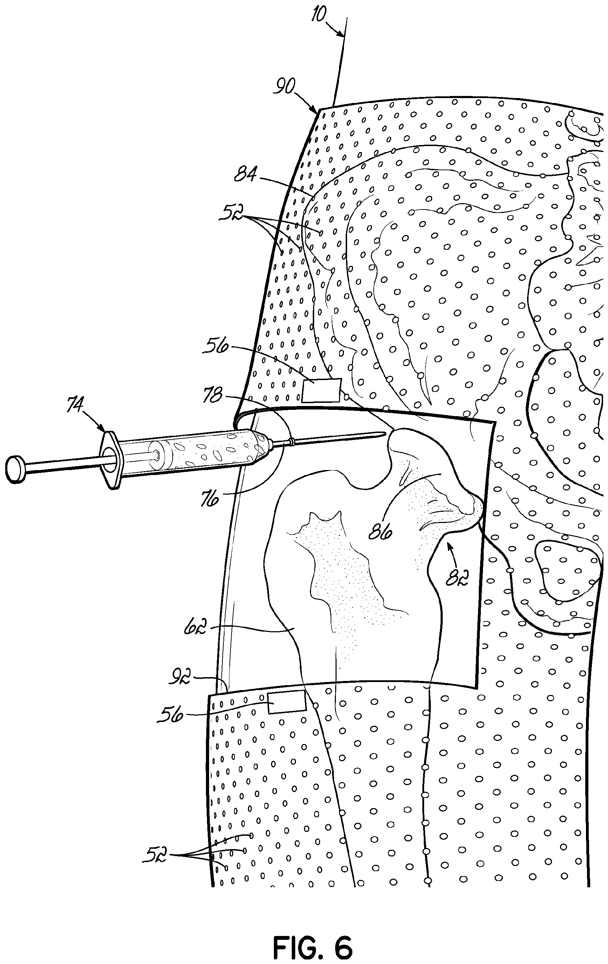

Referring now to FIGS. 5 and 6, an alternative embodiment of the invention is presented that is configured for use with injections into a hip joint. FIG. 5 illustrates a view of a 3-D representation 80 of a hip joint 82 of patient 10. The 3-D representation may be generated as described above with respect to FIGS. 3 and 4, and includes a pelvis 84 and a femoral head 86 of the femur 62. An outline 88 of a drape 90 in accordance with the alternative embodiment is also illustrated showing a window 92 of drape 90 positioned over an area of the patient 10 through which an injection is to be provided to the joint 82. The outline thereby indicates a typical position of the drape 90 relative to the joint 82 during the injection procedure.

The drape 90 includes sensors, which includes one or more ultrasound sensors 52 and one or more IMU sensors 56. In the exemplary embodiment illustrated in FIG. 6, the drape 90 includes a plurality of ultrasound sensors 52 and two IMU sensors 56. Although not illustrated in FIG. 6, the drape 90 may include wires 58 that couple the one or more ultrasound sensors 52 and the one or more IMU sensors 56 to the connector 31, or some other suitable interface that couples the sensors 52, 56 to the ultrasound system 18 and/or computer 22, such as a wireless interface. The ultrasound sensors 52 are configured to be coupled to the ultrasound system 18 to provide ultrasound signals to the ultrasound imaging system 18. These ultrasound signals may be processed to locate the contours of and/or image the bones 62, 84, 86 of joint 82. The computer 22 may then correlate these bone contours or images to the 3-D representation 80, and generate an image 17 based on the 3-D representation of joint 82 in a similar manner as described above with respect to FIGS. 3 and 4.

The IMU sensors 56 are located superior and inferior to the joint 82 to provide positional data to the computer 22 regarding the orientation of the joint 82. This positional data may be used to update the orientation of the 3-D representation 80 of joint 82 in a similar manner as described above with respect to FIG. 4. The sensors 52, 56 may be further configured so that the position of the needle 76 relative to the joint 82 can be determined by the computer 22 as the needle 76 is inserted into the intracapsular space and joint 82. The computer 22 may then include the needle 76 in the image 17 to provide guidance during the injection procedure, which may be an intra-articular injection.

Referring now to FIG. 7, in those instances where the patient 10 moves during the injection procedure, it may be desirable re-localize the position of the needle 76 relative to the new joint position by scanning the joint 82 with ultrasound while the drape is placed on the patient 10. To provide another exemplary method of monitoring and re-localization of the needle 76 relative to the new joint position, it may further be desirable to scan the joint 82 through the drape 90 and the window 92 using the ultrasound probe 96. That is, to scan the joint 82 with the ultrasound probe 96 without removing the drape 90. One such scanning modality may include using a B-mode ultrasound probe 96 having a transducer array 98 for imaging the joint 82. To re-localize the needle 76, pulse echo signals from the ultrasound probe 96 may be used directly to automatically extract bone contours from ultrasound scans and image the joint 82. The extracted bone contours may in turn be used to generate images and/or 3-D models or representations based on the ultrasound RF signal data. Exemplary methods of generating images and 3-D models from pulse echo signals are described in more detail in U.S. Publication No. 2010/0198067, having a filing date of 2 February 2009 and entitled NONINVASIVE DIAGNOSTIC SYSTEM, and in International Application No. PCT/US12/60261, filed on October 15, 2012 and entitled REAL-TIME 3-D ULTRASOUND RECONSTRUCTION OF KNEE AND ITS IMPLICATIONS FOR PATIENT SPECIFIC IMPLANTS AND 3-D JOINT INJECTIONS (Pub. No. WO 2013/056231).

Scanning will typically be performed by placing the ultrasound probe 96 over the window 92, but the probe 96 may also be placed generally or partially over the material comprising the drape 90. Placement over the drape 90 may be used in particular for drapes having a small window 92, or that lack the window 92. That is, embodiments in which the injection is made through the material comprising the drape 90.

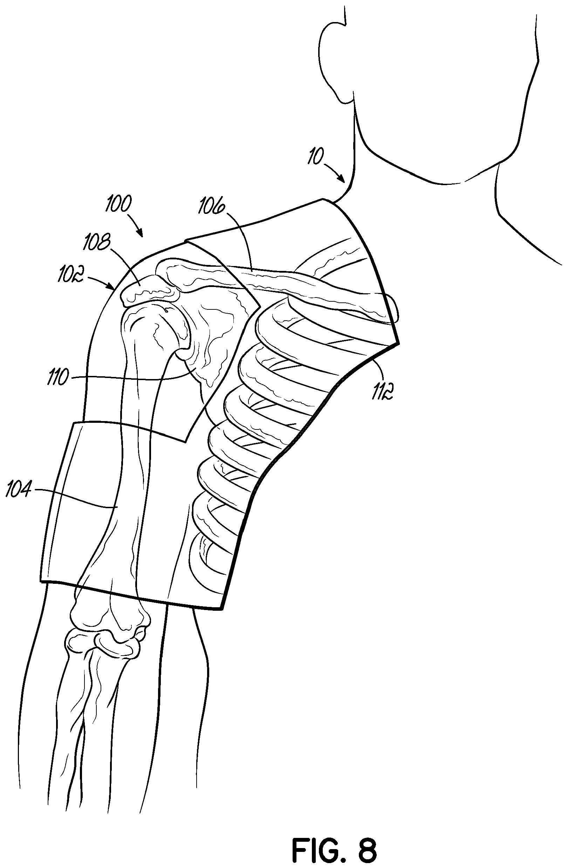

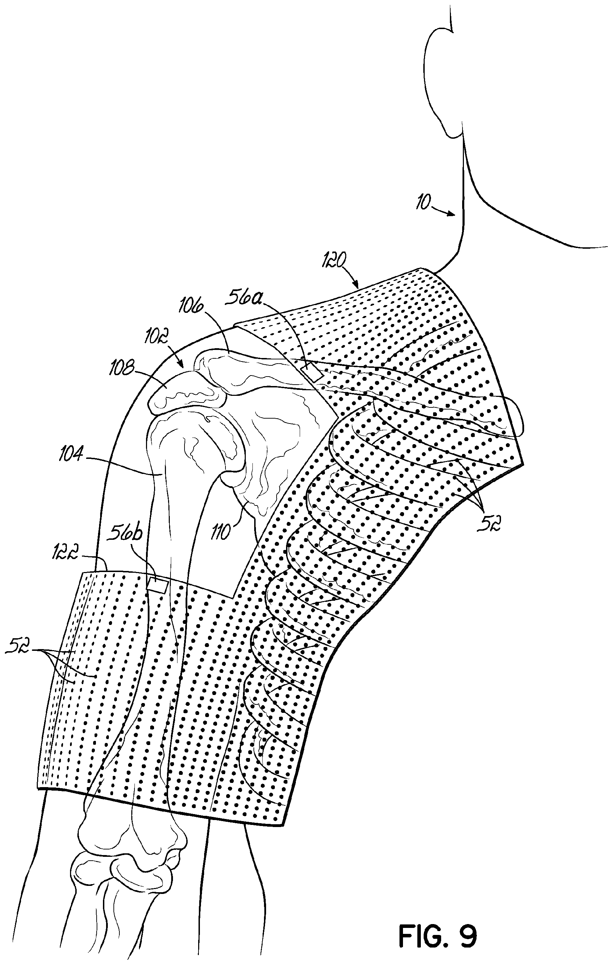

Referring now to FIGS. 8 and 9, FIG. 8 illustrates a 3-D representation 100 of a shoulder joint 102 including a humerus 104, a clavicle 106, and an acromion process 108 of a scapula 110, positioned and ready for an intraarticular (or glenohumeral) or subacromial injection. An outline 112 of a drape 120 in accordance with an alternative embodiment of the invention shows the position of the drape 120 with a window 122 located over the joint 102 to provide access to the joint 102. The outline 112 thereby indicates a typical position of the drape 120 relative to the joint 102 during the injection procedure. As described above, the 3-D representation 100 of the joint 102 may be generated using any suitable medical imaging modalities and stored as digital file on computer 22 prior to the injection procedure.

Similarly as described with respect to the drape 90 used when injecting hip joints, the shoulder drape 120 includes sensors, which includes one or more ultrasound sensors 52 and one or more IMU sensors 56. Although not illustrated in FIG. 9, the drape 120 may include wires 58 that couple the one or more ultrasound sensors 52 and the one or more IMU sensors 56 to the connector 31, or some other suitable interface that couples the sensors 52, 56 to the ultrasound system 18 and/or computer 22, such as a wireless interface. In the exemplary embodiment illustrated in FIG. 9, the drape 120 includes a superior IMU sensor 56a and an inferior IMU sensor 56b. The IMU sensors 56a, 56b are thereby configured to detect motion of the joint 102, and provide signals to the computer 22 for localization of the underlying bones 104, 106, 108, 110 comprising the joint 102 in real time. The ultrasound sensors 52 are configured within the drape 120 to provide ultrasound signals to the ultrasound imaging system 18. These ultrasound signals may be processed to locate the contours of the bones 104, 106, 108, 110 of joint 102. The computer 22 may then correlate these bone contours to the 3-D representation 100, and generate an image 17 based on the 3-D representation 100 of joint 102 in a similar manner as described above with respect to FIGS. 3 and 4.

Similarly to the hip joint drape 90, the IMU sensors 56 in the shoulder drape 120 are located superior and inferior to the joint 102 to provide positional data to the computer 22 regarding the orientation of the joint 102. This positional data may be used to update the orientation of the 3-D representation 100 of joint 102 in a similar manner as described above with respect to FIG. 4. The sensors 52, 56 may be further configured so that the position of the needle 76 relative to the joint 102 can be determined by the computer 22 as the needle 76 is inserted into the intracapsular space and joint 102. The computer may then include a representation of the needle 76 in the image 17 with the 3-D representation 100 of joint 102 to provide guidance during an intra-articular injection. As with the previously described drapes 12, 90, the window 122 of drape 120 provides a sterile field for injection as well as an area that may be utilized for rescanning - if necessary - to re-localize the bones 104, 106, 108, 110 of joint 102.

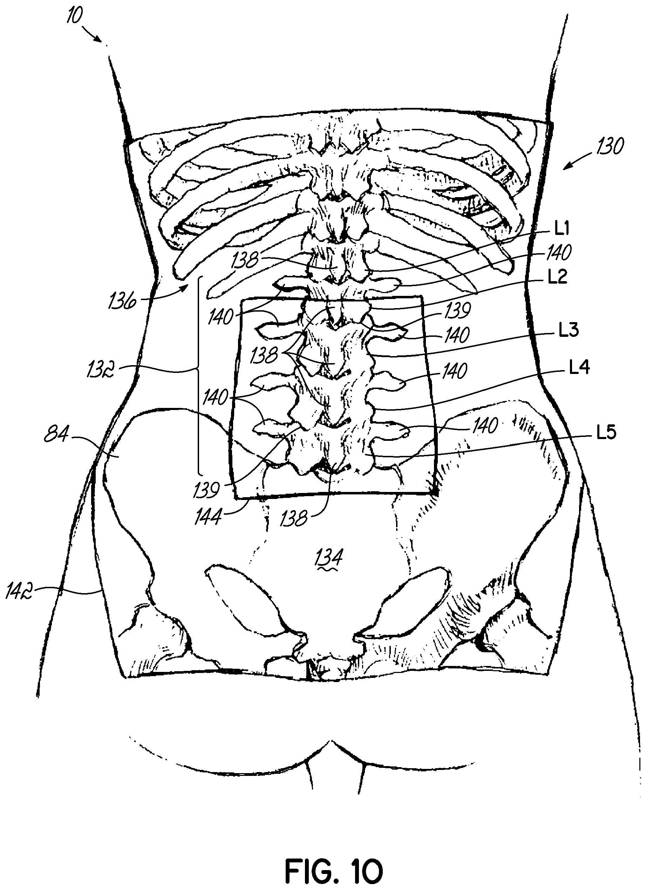

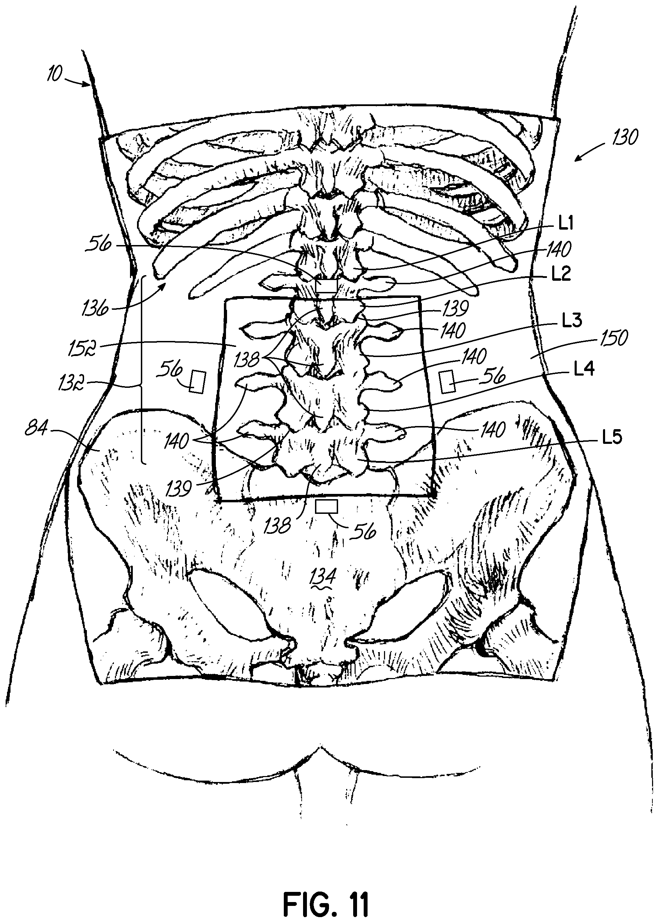

With reference now to FIGS. 10-14, FIG. 10 illustrates a 3-D representation 130 of a lumbar spine 132 of the patient 10. The 3-D representation 130 may include lumbar vertebrae L1-L5, a sacrum 134, and a rib cage 136. Each lumbar vertebrae L1-L5 includes a spinous process 138, a facet joint 139, and a laterally extending transverse processes 140. An outline 142 illustrates the location of a drape 150 (FIG. 11) relative to the lumbar spine 132. The location of a window 152 (FIG. 11) of drape 150 relative to the lumbar spine 132 is similarly indicated by an outline 144. As with the previous drapes 12, 90, 120, the outlines 142, 144 may be superimposed on the image 17 of the 3-D representation 130 to provide the physician with a visual reference during the injection.

Referring now to FIG. 11, the drape 150, which does not form part of the invention, may be configured to support injection of the facet joints 139 and/or an injection within the epidural space of the spine. To this end, the window 152 may be configured to expose a surface region of the patient 10 proximate to the lumbar spine 132. The exemplary drape 150 illustrated in FIG. 11 includes a plurality of IMU sensors 56 configured to detect motion of the patient 10 associated with movement of the spine, and in particular, movement of the lumbar vertebrae L1-L5. In the specific drape shown, four IMU sensors 56 are positioned in a circumferential pattern around the window 152 so that one sensor 56 is located on each side of the window 152. That is, one sensor 56 is placed along each of the left lateral, right lateral, superior (e.g., anterior), and inferior (e.g., posterior) portions of the drape 150 proximate the lumbar spine 132. The IMU sensors 56 are thereby configured to provide signals to the computer 22 indicative of changes in the positions of lumbar vertebrae L1-L5. These signals may be used by the computer 22 to track motion and/or determine whether motion is occurring, and to update the orientation of the lumbar vertebrae L1-L5, the sacrum 134, and the rib cage 136 in the 3-D representation 130.

To determine the initial orientation of the 3-D representation 130, the lumbar spine 132 may be localized by scanning within the window 152 of drape 150, and registering the 3-D representation 130 to the actual orientation of the lumbar spine 132 as indicated by the scan. Once the 3-D representation 130 has been localized to the actual orientation of the lumbar spine 132, the window 152 may be re-sterilized to remove any contamination resulting from the scanning process. Localizing the lumbar spine 132 through the window 152 of drape 150 allows the localization to be made after the IMU sensors 56 are in place so that the initial position of the lumbar spine 132 can be established. This enables tracking of the lumbar spine 132 from its initial position based on motion detected by the IMU sensors 56. Typically, the localization process is performed prior to beginning the injection procedure, although the lumbar spine 132 may be re-localized during the procedure by rescanning if necessary. The lumbar spine 132 may also be localized by scanning the patient 10 prior to placing the drape 12 on the patient.

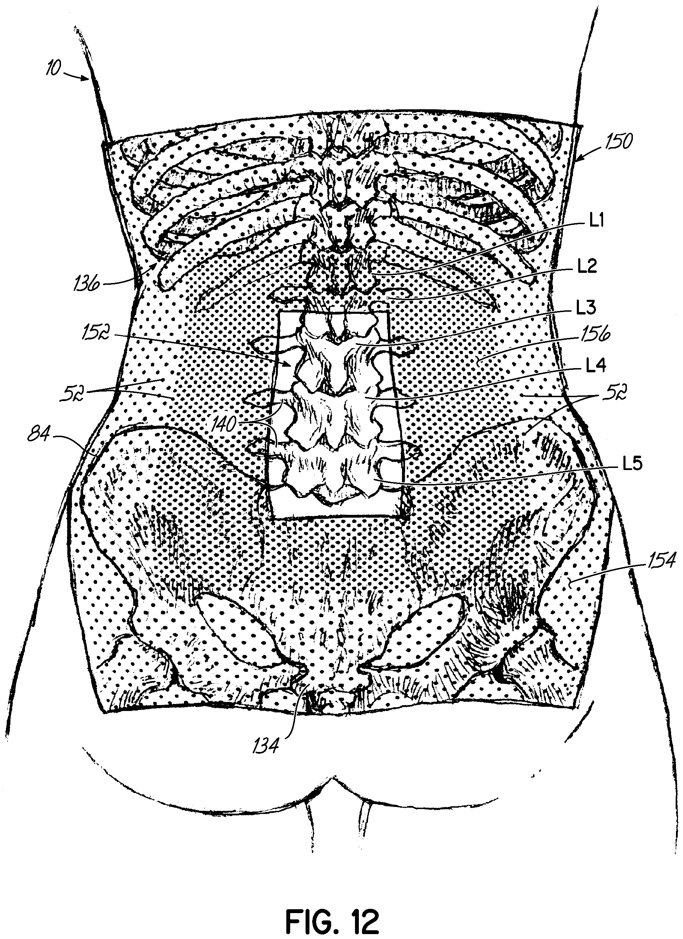

Referring now to FIG. 12, the drape 150, which does not form part of the invention, may include a plurality of ultrasound sensors 52 distributed so that different regions of the drape 150 have different sensor densities. For example, in the illustrated example, the drape 150 is shown with a region 154 having ultrasound sensors 52 arranged with relatively low density, and a region 156 having ultrasound sensors 52 arranged with relatively high density. That is, the average distance between adjacent ultrasound sensors 52 in region 154 of drape 150 is greater than the average distance between adjacent ultrasound sensors 52 in region 156 of drape 150. The region 156 having the higher density of sensors 52 may be positioned proximate the window 152 to enhance the imaging capabilities in that region of the patient 10. The drape 150 may thereby provide improved visualization of the specific area where the injection is to occur as compared to drapes lacking the high density region 156. Although not illustrated in FIG. 12, the drape 150 may also include the IMU sensors 56 shown in FIG. 11 in addition to the ultrasound sensors 52. Moreover, these sensors may be arranged into more than two regions having different sensor densities.

Although not illustrated in FIG. 12 the drape 120 may also include wires 58 that couple the one or more ultrasound sensors 52 and the one or more IMU sensors 56 to the connector 31, or some other suitable interface that couples the sensors 52, 56 to the ultrasound system 18 and/or computer 22, such as a wireless interface. In an alternative embodiment of the invention having the wireless interface, the interface may include wireless data transmission circuitry (not shown) configured to put the sensors 52, 56 in communication with the ultrasound system 18 and/or computer 22, thereby avoiding the need for the cable 28.

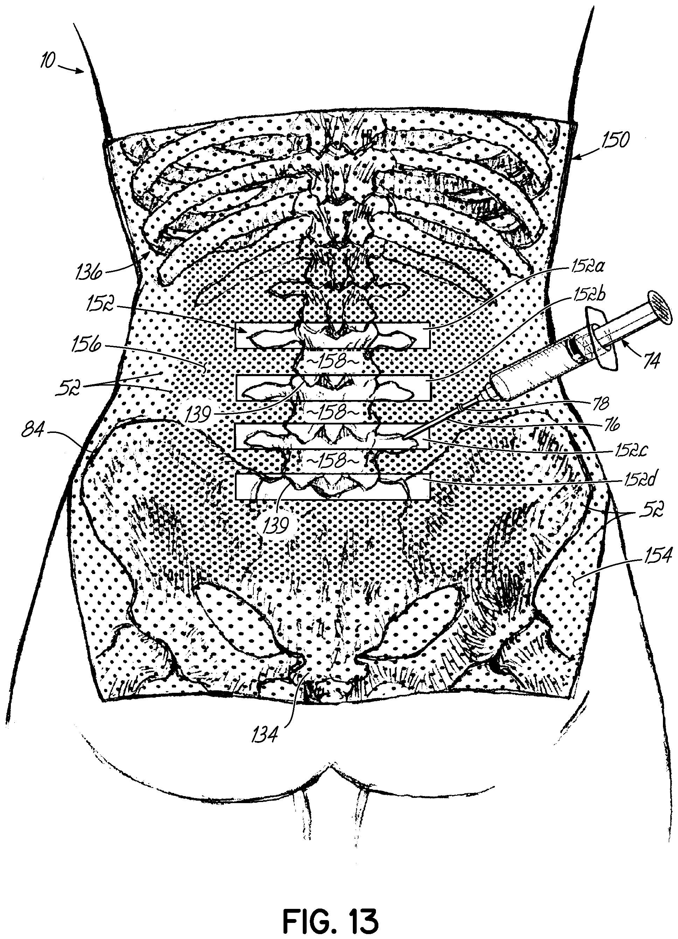

FIG. 13 illustrates the drape 150, which does not form part of the invention, configured for use with injections of the lumbar spine 132 that has a plurality of windows 152a-152d. The windows 152a-152d may be configured to provide openings in the drape 150 that correspond to positions of the facet joints 139 between the lumbar vertebrae L1-L5 and the sacrum 134. The plurality of windows 152a-152d may thereby define a plurality of sections 158 of drape 150 that extend across the lumbar spine 132. These sections 158 of drape 150 may in turn enable additional ultrasound and/or IMU sensors 52, 56 to be placed in closer proximity to regions of the lumbar spine 132 that are to be injected as compared to drapes 150 lacking the sections 158.

Similarly to the drape illustrated in FIG. 12, the exemplary drape 150 illustrated in FIG. 13 includes regions 154, 156 having ultrasound sensors arranged with different densities. The region 156 of drape 150 having the higher ultrasound sensor density may include the sections 158 of drape 150. The additional ultrasound sensors 52 provided by the sections 158 of drape 150 extending across the lumbar spine 132 may further enhance the imaging capabilities as compared to the embodiment illustrated in FIG. 12. This may improve visualization of the lumbar spine 132, and more specifically, areas exposed by the windows 152a-152d for injection by needle 76 of syringe 74. The increased density of ultrasound sensors 52 in region 156 may also decrease or eliminate the need for motion sensors, such as the IMU sensors 56 shown in FIG. 11.

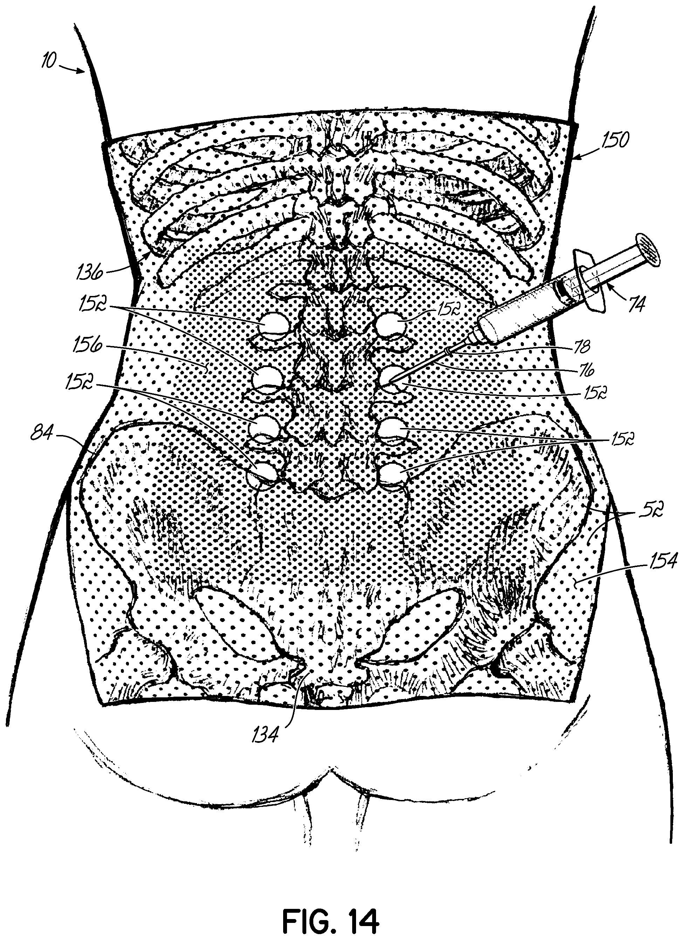

FIG. 14 illustrates another drape, which does not form part of the invention, configured for use with the lumbar spine 132 that has a plurality of windows 152 arranged in two columns generally parallel with the lumbar spine 132. The windows 152 are defined by a plurality of openings in the drape 150 that are aligned for sterile injection, such as for lateral facet injections. Use of the exemplary drape illustrated in FIG. 14 may be preferred when the lumbar spine 132 is well visualized. That is, a well visualized lumbar spine 132 may allow the windows 152 to be aligned with the desired injection points with sufficient accuracy so that only a small opening is needed for injecting the underlying joint. By exposing only a small area over the injection point, the configuration of the windows 152 may permit additional ultrasound sensors 52 to be located in close proximity to the injection point. These additional sensors 52 may provide further improvements in the quality of imaging data provided to the computer 22. This improved imaging data may in turn result in improved accuracy of the orientation of the lumbar spine 132, as well as the location of needle 76 displayed by the image 17 on monitor 26.

In the illustrated drape, the openings defining the windows 152 are shown placed along the spinous processes 138. In other exemplary drapes, such as for those suitable for use in an epidural injection, the openings may be positioned proximate the midline to access the epidural space. As described above with respect to FIGS. 12 and 13, the drape 150 may include multiple regions 154, 156 having different sensor densities to increase the image resolution capabilities proximate the windows 152, and configured to aid in the proper alignment of the needle 76.

While the present invention has been illustrated by a description of various illustrative embodiments, and while these embodiments have been described in some detail, it is not the intention of the Applicants to restrict or in any way limit the scope of the appended claims to such detail. Additional advantages and modifications will readily appear to those skilled in the art. For example, although the windows 14, 92, 122, 152 are shown as having either a generally rectangular or circular shape, persons having ordinary skill in the art would understand that the windows 14, 92, 122, 152 may be shaped and sized to accommodate any size injection point or to accommodate any procedure. The configuration of the windows 14, 92, 122, 152 are therefore not limited the particular shapes and sizes illustrated herein.

Persons having ordinary skill in the art will further understand that each embodiment of the drapes described herein could have any combination of: (1) different densities and/or numbers of ultrasound sensors in different regions or portions of the drape, (2) different numbers, patterns, and/or densities of embedded IMU sensors, (3) sensors configured to provide a scanning capability in the injected area to re-localize the joint and/or needle to the 3-D representation, and/or (4) an area configured for scanning through the drape material for either re-localization of the underlying bones or during injection for higher accuracy needle placement. In addition, each embodiment may include sections of the drape that permit penetration by a needle to provide the ability to inject through the drape. Moreover, these sections may also provide at least some visibility of the underlying surface of the patient.

Embodiments of the invention include embodiments of the drapes 12, 90, 120, 150 that are configured so that the respective joint or joints 16, 82, 102, 132 can be scanned through the drape 12, 90, 120, 150 and the corresponding 3-D representation 60, 80, 100, 130 registered to its corresponding joint or joints by the computer 22. These embodiments include IMU sensors 56 that can be used to monitor motion during the injection and through the silicone drape material.

Persons having ordinary skill in the art will also understand that embodiments of the invention include drapes 12, 90, 120, 150 that include both ultrasound sensors 52 and IMU sensors 56. Moreover, persons having ordinary skill in the art will further understand that these sensors may be arranged into one region having a sensor density, or a plurality of regions, with each region having a different sensor density.

In addition, the various features of the invention may be used alone or in any combination depending on the needs and preferences of the user. However, the invention itself should only be defined by the appended claims.

## Claims

1. A guided needle injection system comprising: a drape (12) configured to be positioned over a joint (16) of a patient (10), the joint having an orientation; and
one or more ultrasound sensors (52) and one or more inertial measurement units (56) coupled to the drape and configured to provide signals indicative of the orientation of the joint.

2. The system of claim 1 wherein the signals indicative of the orientation of the joint (16) indicate a change in a position of the patient (10).

3. The system of claim 1 wherein a change in the orientation of the joint (16) is indicated by a change in a determined position of a bone contour.

4. The system of claim 1 wherein the one or more ultrasound sensors (52) is configured to scan at least one of a contour of a bone (62, 64, 66, 68) comprising the joint (16) and a position of a needle (76) inserted into the joint (16).

5. The system of claim 1 wherein the one or more ultrasound sensors (52) includes a first plurality of ultrasound sensors distributed over a first portion of the drape (12) at a first density, and a second plurality of ultrasound sensors (52) distributed over a second portion of the drape (12) at a second density different than the first density.

6. The system of claim 5 wherein the second portion of the drape (12) is positioned closer to an injection window (14) than the first portion of the drape.

7. The system of claim 1 further comprising:
a window (14) in the drape (12), the window (14) configured to be located over a desired injection site of the patient (10) in response to the drape (12) being placed on the patient.

8. The system of claim 7 wherein the window (14) is comprised of a material that is transparent to ultrasound.

9. The system of claim 8 wherein the window (14) has a first surface configured to contact the patient and a second surface opposite the first surface, the second surface configured to be scanned by a sensor (96) that is external to the medical drape (12).

10. The system of claim 8 wherein the window (14) includes an opening positioned over the injection site.

11. The system of claim 7 wherein the window (14) is comprised of an opening in the drape (12).

12. The system of claim 1 further comprising:
a 3-D representation (60) of a patient (10) joint (16) having an orientation, the orientation of the 3-D representation (60) of the joint (16) being stored in a memory (42) and adjusted based on the signals indicative of the orientation of the joint (16) provided by the one or more ultrasound sensors (52) and the one or more inertial measurement units (56) of the drape (12).

13. The system of claim 12 wherein the orientation of the 3-D representation (60) of the joint (16) is adjusted based on the signals provided by the one or more inertial measurement units (56) indicating a change in the position of the one or more inertial measurement units (56) from an initial position.

14. The system of claim 12, wherein the orientation of the 3-D representation (60) of the joint (16) is adjusted based on the signals provided by the one or more ultrasound sensors (52) indicating a position of a bone contour corresponding to the joint in the 3-D representation (60).

15. The system of claim 1 further comprising:
a computer (22) coupled to the one or more ultrasound sensors (52) and to the one or more inertial measurement units (56) to receive signals indicative of the
orientation of the joint (16), the computer (22) configured to register a three dimensional representation (60) of the joint (16) and generate an image (17) based on the three dimensional representation to be displayed and updated in real-time based on the orientation of the joint (16); and,
a monitor (26) coupled to the computer (22) and configured to display the image (17).

## Patentansprüche

1. Geführtes Nadelinjektionssystem, umfassend: eine Abdeckung (12), die konfiguriert ist, um über ein Gelenk (16) eines Patienten (10) positioniert zu werden, wobei das Gelenk eine Ausrichtung aufweist; und
einen oder mehrere Ultraschallsensoren (52) und eine oder mehrere Inertialmesseinheiten (56), die mit der Abdeckung gekoppelt und konfiguriert sind, um Signale bereitzustellen, die die Ausrichtung des Gelenks anzeigen.

2. System nach Anspruch 1, wobei die Signale, die die Ausrichtung des Gelenks (16) anzeigen, eine Änderung der Position des Patienten (10) anzeigen.

3. System nach Anspruch 1, wobei eine Änderung der Ausrichtung des Gelenks (16) durch eine Änderung einer bestimmten Position einer Knochenkontur angezeigt wird.

4. System nach Anspruch 1, wobei der eine oder die mehreren Ultraschallsensoren (52) konfiguriert ist, um mindestens eine einer Kontur eines Knochens (62, 64, 66, 68), umfassend das Gelenk (16), und einer Position einer in das Gelenk (16) eingeführten Nadel (76) zu scannen.

5. System nach Anspruch 1, wobei der eine oder die mehreren Ultraschallsensoren (52) eine erste Vielzahl von Ultraschallsensoren enthält, die über einen ersten Abschnitt der Abdeckung (12) in einer ersten Dichte verteilt sind, und eine zweite Vielzahl von Ultraschallsensoren (52), die über einen zweiten Abschnitt der Abdeckung (12) in einer zweiten Dichte, die sich von der ersten Dichte unterscheidet, verteilt sind.

6. System nach Anspruch 5, wobei der zweite Abschnitt der Abdeckung (12) näher an einem Injektionsfenster (14) positioniert ist als der erste Abschnitt der Abdeckung.

7. System nach Anspruch 1, ferner umfassend:
ein Fenster (14) in der Abdeckung (12), wobei das Fenster (14) konfiguriert ist, um in Reaktion darauf, dass die Abdeckung (12) auf dem Patienten platziert wird, über einer erwünschten Injektionsstelle des Patienten (10) lokalisiert zu werden.

8. System nach Anspruch 7, wobei das Fenster (14) aus Material besteht, das gegenüber Ultraschall transparent ist.

9. System nach Anspruch 8, wobei das Fenster (14) eine erste Oberfläche, die konfiguriert ist, um in Kontakt zum Patienten zu stehen, und eine zweite Oberfläche gegenüber der ersten Oberfläche aufweist, wobei die zweite Oberfläche konfiguriert ist, um von einem Sensor (96) gescannt zu werden, der extern von der medizinischen Abdeckung (12) ist.

10. System nach Anspruch 8, wobei das Fenster (14) eine über der Injektionsstelle positionierte Öffnung enthält.

11. System nach Anspruch 7, wobei das Fenster (14) aus einer Öffnung in der Abdeckung (12) besteht.

12. System nach Anspruch 1, ferner umfassend:
eine 3D-Darstellung (60) eines Gelenks (16) eines Patienten (10) mit einer Ausrichtung, wobei die Ausrichtung der 3D-Darstellung (60) des Gelenks (16) in einem Speicher (42) gespeichert und auf der Grundlage der Signale, die die Ausrichtung des Gelenks (16) anzeigen und von dem einem oder den mehreren Ultraschallsensoren (52) und der einen oder den mehreren Inertialmesseinheiten (56) der Abdeckung (12) bereitgestellt werden, angepasst wird.

13. System nach Anspruch 12, wobei die Ausrichtung der 3D-Darstellung (60) des Gelenks (16) auf der Grundlage der Signale angepasst wird, die von der einen oder den mehreren Inertialmesseinheiten (56) bereitgestellt werden, die eine Änderung der Position der einen oder der mehreren Inertialmesseinheiten (56) gegenüber einer Ausgangsposition anzeigen.

14. System nach Anspruch 12, wobei die Ausrichtung der 3D-Darstellung (60) des Gelenks (16) auf der Grundlage der Signale angepasst wird, die von dem einen oder den mehreren Ultraschallsensoren (52) bereitgestellt werden, die eine Position einer Knochenkontur anzeigen, die dem Gelenk in der 3D-Darstellung (60) entsprechen.

15. System nach Anspruch 1, ferner umfassend:
einen Computer (22), der mit dem einen oder den mehreren Ultraschallsensoren (52) und mit der einen oder den mehreren Inertialmesseinheiten (56) gekoppelt ist, um Signale zu empfangen, die die Ausrichtung des Gelenks (16) anzeigen, wobei der Computer (22) konfiguriert ist, eine dreidimensionale Darstellung (60) des Gelenks (16) zu registrieren und ein Bild (17) auf der Grundlage der dreidimensionalen Darstellung zur Anzeige und Echtzeit-Aktualisierung auf der Grundlage der Ausrichtung des Gelenks (16) zu erzeugen; und
einen Monitor (26), der mit dem Computer (22) gekoppelt und konfiguriert ist, das Bild (17) anzuzeigen.

## Revendications

1. Système d'injection par aiguille guidé comprenant : un champ opératoire (12) conçu pour être placé sur une articulation (16) d'un patient (10), l'articulation présentant une orientation ; et
un ou plusieurs capteurs à ultrasons (52) et une ou plusieurs unités de mesure inertielle (56) couplées au champ opératoire et conçues pour fournir des signaux indiquant l'orientation de l'articulation.

2. Système selon la revendication 1, lesdits signaux indiquant l'orientation de l'articulation (16) indiquant un changement de position du patient (10).

3. Système selon la revendication 1, un changement de l'orientation de l'articulation (16) étant indiqué par un changement dans une position déterminée d'un contour d'os.

4. Système selon la revendication 1, lesdits un ou plusieurs capteurs à ultrasons (52) étant conçus pour balayer au moins l'un d'un contour d'un os (62, 64, 66, 68) comprenant l'articulation (16) et d'une position d'une aiguille (76) insérée dans l'articulation (16).

5. Système selon la revendication 1, lesdits un ou plusieurs capteurs à ultrasons (52) comprenant une première pluralité de capteurs à ultrasons répartis sur une première partie du champ opératoire (12) à une première densité, et une seconde pluralité de capteurs à ultrasons (52) répartis sur une seconde partie du champ opératoire (12) à une seconde densité différente de la première densité.

6. Système selon la revendication 5, ladite seconde partie du champ opératoire (12) étant placée plus près d'une fenêtre d'injection (14) que la première partie du champ opératoire.

7. Système selon la revendication 1 comprenant en outre :
une fenêtre (14) dans le champ opératoire (12), la fenêtre (14) étant conçue pour se trouver sur un site d'injection souhaité du patient (10) en réponse au champ opératoire (12) placé sur le patient.

8. Système selon la revendication 7, ladite fenêtre (14) étant constituée d'un matériau qui est transparent aux ultrasons.

9. Système selon la revendication 8, ladite fenêtre (14) comportant une première surface conçue pour être en contact avec le patient et une seconde surface opposée à la première surface, la seconde surface étant conçue pour être balayée par un capteur (96) qui est externe au champ opératoire médical (12).

10. Système selon la revendication 8, ladite fenêtre (14) comportant une ouverture se trouvant sur le site d'injection.

11. Système selon la revendication 7, ladite fenêtre (14) étant constituée d'une ouverture dans le champ opératoire (12).

12. Système selon la revendication 1 comprenant en outre :
une représentation 3D (60) d'une articulation (16) de patient (10) présentant une orientation, l'orientation de la représentation 3D (60) de l'articulation (16) étant stockée dans une mémoire (42) et ajustée sur la base des signaux indiquant l'orientation de l'articulation (16) fournie par lesdits un ou plusieurs capteurs à ultrasons (52) et lesdites une ou plusieurs unités de mesure inertielle (56) du champ opératoire (12).

13. Système selon la revendication 12, ladite orientation de la représentation 3D (60) de l'articulation (16) étant ajustée sur la base des signaux fournis par lesdites une ou plusieurs unités de mesure inertielle (56) indiquant un changement de position desdites une ou plusieurs unités de mesure inertielle (56) depuis une position initiale.

14. Système selon la revendication 12, ladite orientation de la représentation 3D (60) de l'articulation (16) étant ajustée sur la base des signaux fournis par lesdits un ou plusieurs capteurs à ultrasons (52) indiquant une position d'un contour d'os correspondant à l'articulation dans la représentation 3D (60).

15. Système selon la revendication 1 comprenant en outre :
un ordinateur (22) couplé auxdits un ou plusieurs capteurs à ultrasons (52) et auxdites une ou plusieurs unités de mesure inertielle (56) pour recevoir des signaux indiquant l'orientation de l'articulation (16), l'ordinateur (22) étant conçu pour enregistrer une représentation tridimensionnelle (60) de l'articulation (16) et générer une image (17) sur la base de la représentation tridimensionnelle à afficher et mettre à jour en temps réel sur la base de l'orientation de l'articulation (16) ; et
un moniteur (26) couplé à l'ordinateur (22) et conçu pour afficher l'image (17).
